(19) Europäisches Patentamt
European Patent Office
Office européen des brevets

(11) Veröffentlichungsnummer: **0 485 929 A1**

(12) # EUROPÄISCHE PATENTANMELDUNG

(21) Anmeldenummer: **91119186.4**

(22) Anmeldetag: **11.11.91**

(51) Int. Cl.5: **C07D 233/68**, C07D 233/70, C07D 413/10, A61K 31/415, C07D 403/10, A61K 31/42

(30) Priorität: **16.11.90 DE 4036645**

(43) Veröffentlichungstag der Anmeldung:
**20.05.92 Patentblatt 92/21**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kleemann, Heinz-Werner, Dr.**
**An der hohlen Eiche 3**
**W-6380 Bad Homburg(DE)**
Erfinder: **Gerhards, Hermann, Dr.**
**Wacholderweg 4**
**W-6238 Hofheim am Taunus(DE)**
Erfinder: **Schölkens, Bernward, Prof. Dr.**
**Hölderlinstrasse 62**
**W-6233 Kelkheim/Taunus(DE)**

(54) **Substituierte Azole, Verfahren zu ihrer Herstellung und ihre Verwendung.**

(57) Azole der Formel

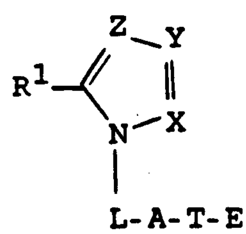

worin X, Y und Z für N oder $CR^2$ stehen, L Alkylen, A einen aromatischen oder heterocyclischen Rest und T eine Bindung oder einen bivalenten Rest bedeuten und $R^1$, $R^2$ und E wie in der Beschreibung definiert sind.

Weiter werden Verfahren zu ihrer Herstellung, diese Verbindungen enthaltende Mittel und ihre Verwendung als Heilmittel beschrieben.

EP 0 485 929 A1

Aus EP-A-324377, EP-A-253310, EP-A-28834 und EP-A 323841 sind Derivate von Imidazol, Pyrrol, Pyrazol bzw. Triazol und deren Verwendung als Antagonisten von Angiotensin-II-Rezeptoren bekannt.

Es wurden nun neue Verbindungen vom Azol-Typ gefunden, die überraschenderweise hochwirksame Antagonisten von Angiotensin-II-Rezeptoren sowohl in vitro als in vivo sind.

Die Erfindung betrifft Verbindungen der Formel I,

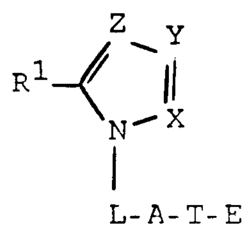

(I)

in welcher

a) X, Y und Z gleich oder verschieden sind und N oder $CR^2$ bedeuten,

b)

$R^1$

1. $(C_2\text{-}C_{10})$-Alkyl,
2. $(C_3\text{-}C_{10})$-Alkenyl,
3. $(C_3\text{-}C_{10})$-Alkinyl,
4. $OR^3$,
5. $(C_3\text{-}C_8)$-Cycloalkyl,
6. $(C_4\text{-}C_{10})$-Cycloalkylalkyl,
7. $(C_5\text{-}C_{10})$-Cycloalkylalkenyl,
8. $(C_5\text{-}C_{10})$-Cycloalkylalkinyl,
9. $-(CH_2)_m\text{-}B\text{-}(CH_2)_n\text{-}R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1\text{-}C_4)$-Alkoxy und Nitro substituiert ist,

bedeutet;

c)

$R^2$

1. Wasserstoff,
2. Halogen,
3. Nitro,
4. $C_vF_{2v+1}$,
5. $SF_5$,
6. Pentafluorphenyl,
7. Cyano,
8. $(C_1\text{-}C_4)$-Alkoxy, Benzyloxy,
9. Phenyl,
10. Phenyl-$(C_1\text{-}C_3)$-alkyl,
11. $(C_1\text{-}C_{10})$-Alkyl,
12. $(C_3\text{-}C_{10})$-Alkenyl,
13. Phenyl-$(C_2\text{-}C_6)$-Alkenyl,
14. 1-Imidazolyl-$(CH_2)_m$-,
15. 1,2,3-Triazolyl-$(CH_2)_n$-,
16. Tetrazolyl-$(CH_2)_m$-,
17. $-(CH_2)_{o\text{-}1}\text{-}CHR^7\text{-}OR^5$,
18. $-(CH_2)_o\text{-}O\text{-}CO\text{-}R^3$,
19. $-(CH_2)_o\text{-}S\text{-}R^6$,
20. $-S(O)_r\text{-}R^6$,
21. $-CH=CH\text{-}(CH_2)_m\text{-}CHR^3\text{-}OR^6$,

22. $-CH_2=CH-(CH_2)_m-CO-R^8$,
23. $-CO-R^8$,
24. $-CH=CH-(CH_2)_m-O-CO-R^7$,
25. $-(CH_2)_m-CH(CH_3)-CO-R^8$,
26. $-(CH_2)_o-CO-R^8$,
27.

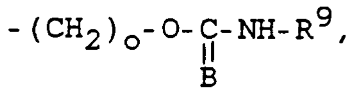

28.

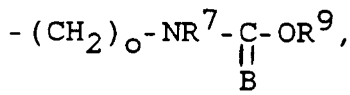

29. $-(CH_2)_o-NR^7-CO-NHR^9$
30. $-(CH_2)_o-NR^7-SO_2R^9$,
31.

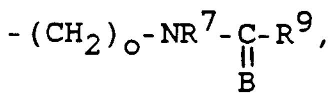

32. $-(CH_2)_nF$,
33. $-(CH_2)_n-O-NO_2$,
34. $-CH_2-N_3$,
35. $-(CH_2)_n-NO_2$,
36. $-CH=N-NR^5R^7$,
37. Phthalimido-$(CH_2)_n$-,
38.

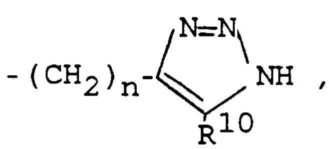

39.

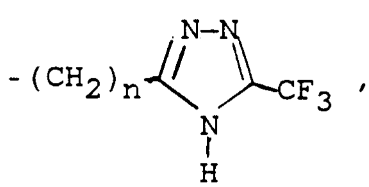

40.

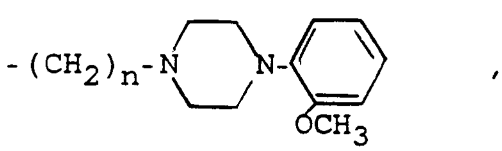

41.

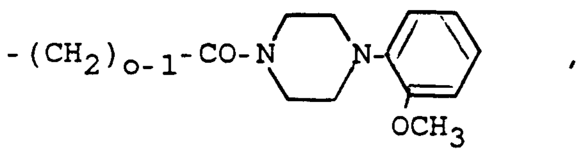

42. Phenyl-$SO_2$-NH-N=CH-,
43.

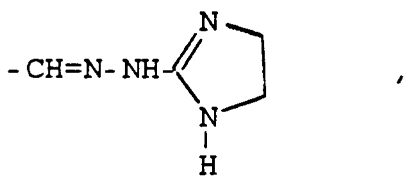

44. -$(CH_2)_n$-$SO_2$-$NR^7$-CO-$NR^6R^9$,
45. -$(CH_2)_o$-$SO_2R^9$,
46. einem wie unter c) 9. oder 10. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
47. einem wie unter c) 11., 12. oder 20. definierten Rest, worin 1 bis alle H-Atome durch Fluor substituiert sind, oder
48. den unter c) 15. definierten Rest, der mit 1 oder 2 gleichen oder verschiedenen Resten aus der Reihe Methoxycarbonyl und ($C_1$-$C_4$)-Alkyl substituiert ist,

bedeutet;

d)
$R^3$
1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeuten;

e)
$R^4$
1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl

bedeutet;

f)
$R^5$
1. Wasserstoff,
2. ($C_1$-$C_6$)-Alkyl,
3. ($C_3$-$C_8$)-Cycloalkyl,
4. Phenyl oder
5. Benzyl

bedeutet;

g)
$R^6$
1. Wasserstoff,
2. ($C_1$-$C_6$)-Alkyl,
3. ($C_3$-$C_8$)-Cycloalkyl,
4. ($C_6$-$C_{12}$)-Aryl, vorzugsweise Phenyl,

5. Benzyl,
6. $(C_1-C_9)$-Heteroaryl, das auch teilweise oder vollständig hydriert sein kann, vorzugsweise 2-Pyrimidinyl,
7. $(C_1-C_4)$-Alkanoyl,
8. $(C_1-C_9)$-Heteroaryl-$(C_1-C_3)$-alkyl, wobei der Heteroarylteil auch teilweise oder vollständig hydriert sein kann, oder
9. einen wie unter g) 4., 6. oder 8. definierten Rest, substituiert mit 1 oder 2 gleichen oder verschiedenen Resten aus der Reihe Halogen, Hydroxy, Methoxy, Nitro, Cyano, $CO_2R^3$, $-NR^{11}R^{12}$ und Trifluormethyl,

bedeutet;

h)
$R^7$

1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl

bedeutet;

i)
$R^8$

1. Wasserstoff,
2. $(C_1-C_6)$-Alkyl,
3. $(C_3-C_8)$-Cycloalkyl,
4. Phenyl-$(CH_2)_q$-,
5. $OR^5$,
6. $NR^{11}R^{12}$ oder
7.

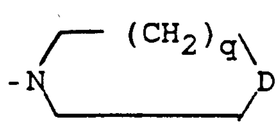

bedeutet;

j)
$R^9$

1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

oder $R^6$ und $R^8$ stehen gemeinsam mit dem diese tragendem N-Atom für

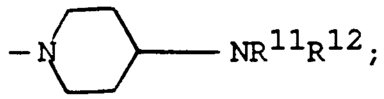

k)
$R^{10}$ Cyano, Nitro oder $CO_2R^7$ bedeutet;

l)
$R^{11}$ und $R^{12}$ gleich oder verschieden sind und

1. Wasserstoff,
2. $(C_1-C_4)$-Alkyl,
3. Phenyl,

4. Benzyl oder
5. $\alpha$-Methylbenzyl

bedeuten;

m) D $NR^{13}$, O oder $CH_2$ bedeutet;

n) $R^{13}$ Wasserstoff, $(C_1-C_4)$Alkyl oder Phenyl bedeutet;

o) A

$\alpha$) einen $(C_6-C_{14})$-Arylrest oder

$\beta$) $(C_1-C_9)$-Heteroaryl, der entweder aromatisch, teilhydriert oder vollständig hydriert sein kann oder

$\gamma$) den Rest eines anellierten Heterocyclus mit 8 bis 10 Ringatomen, wovon bis zu 9 Ringatome C-Atome sind, bedeutet, wobei

A jeweils mit bis zu 3 gleichen oder verschiedenen Resten aus der Reihe

1. Halogen,
2. Oxo,
3. Nitroso,
4. Nitro,
5. Cyano,
6. Hydroxy,
7. $(C_1-C_6)$-Alkyl,
8. $(C_1-C_4)$-Alkanoyl,
9. $(C_1-C_4)$-Alkanoyloxy,
10. $CO_2R^3$,
11. Methansulfonylamino,
12. Trifluormethansulfonylamino,
13. $-CO-NH-OR^9$,
14. $-SO_2-NR^6R^7$,
15. $-CH_2-OR^7$,
16. $(C_6-C_{12})$-Aryl
17. $(C_3-C_8)$-Cycloalkyl,
18. $(C_1-C_4)$-Alkoxy,
19. $(C_1-C_9)$-Heteroaryl,
20. $CO_2R^3$,
21. $NR^6R^7$,
22. Sulfo,
23. $-SO_3R^3$,
24. $-SO_2-NR^7-CO-NR^6R^9$,
25. $-NR^7-CO-NR^6-SO_2-CH_2-R^5$,
26. $-C(CF_3)_2OH$,
27. Phosphonooxy,

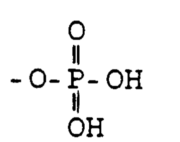 ,

28. $-PO_3H_2$,
29. $-NH-PO(OH)_2$,
30. $-S(O)_rR6$,
31. $-CO-R^8$ und
32. $-CO-NR^6R^9$

substituiert sein kann;

p) T

1. eine Einfachbindung,

2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. -CH = CH-
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

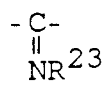 ,

26.

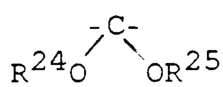

oder
27.

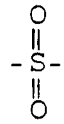

bedeutet;

q)
E einen Rest
1.

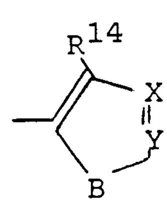

unter der Voraussetzung, daß im Falle X = Y = CH $R^{14}$ nicht COOH bedeutet,

2.

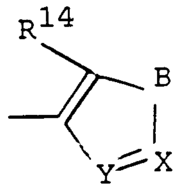

3.

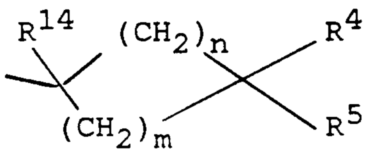

4.

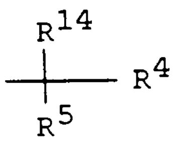

bedeutet;

r)
B O, $NR^7$ oder S bedeutet;

s)
L $(C_1-C_3)$-Alkandiyl bedeutet;

t)
$R^{14}$ $-CO_2R^3$, $-CH_2CO_2R^3$, $-PO_3H_2$, $-SO_3H$ oder Tetrazolyl bedeutet;

u)
m eine ganze Zahl von 0 bis 5 ist,

v)
n eine ganze Zahl von 1 bis 5 ist;

w)
o eine ganze Zahl von 1 bis 10 ist;

x)
r 0, 1 oder 2 ist, und

y)
v eine ganze Zahl von 1 bis 6 ist;

sowie deren physiologisch verträglichen Salze.

Alkyl, Alkenyl und Alkinyl können geradkettig oder verzweigt sein. Entsprechendes gilt für davon abgeleitete Reste, wie Alkanoyl oder Alkoxy.

Unter Cycloalkyl werden auch alkylsubstituierte Ringe verstanden.

$(C_6-C_{12})$-Aryl ist beispielsweise Phenyl, Naphthyl oder Biphenylyl vorzugsweise Phenyl. Entsprechendes gilt für davon abgeleitete Reste, wie Aroyl oder Aralkyl.

Unter $(C_1-C_9)$-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Weiter kann auch 1 oder beide Atome der Kondensationsstelle bicyclischer Reste (wie im Indolizinyl) ein N-Atom sein.

Dies sind z.B. Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Unter dem anelliertem Heterobicyclus versteht man insbesondere ein bicyclisches Ringsystem mit 8 bis 10 Ringatomen, wovon bis zu 9 Ringatome C-Atome sind, worin zwei nebeneinanderliegende Atome gemeinsame Bestandteile beider Ringe sind. Einer oder beide dieser Ringe leiten sich formal vom Benzol ab, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind.

Dies sind beispielsweise ein Rest von Benzothiophen, Benzofuran, Indol, Isoindol, Indazol, Benzimidazol, Chinolin, Isochinolin, Phthalazin, Chinoxalin, Chinazolin, Cinnolin, Benzthiazol, Benzothiazol-1,1-dioxid, Cumarin, Chroman, Benzoxazol, Benzisothiazol, Benzodiazine, Benztriazol, Benztriazin, Benzoxazin. Imidazo-pyridin, Imidazo-pyrimidin, Imidazo-pyrazin, Imidazo-pyridazin, Imidazo-thiazol, Pyrazolopyridin, Thienopyridin und Pyrrolopyrimidin. Der genannte Heterobicyclus kann auch teilweise oder ganz hydriert sein. Vorzugsweise bleibt aber ein Ring aromatisch, wobei ein benzanellierter Heterobicyclus besonders bevorzugt ist.

Im Falle von S-haltigen und/oder teilgesättigten Resten kann der Bicyclus z.B. auch oxosubstituiert sein, wie es beim Rest des Benz-1,2,3-triazinon der Fall ist.

Unter physiologisch verträglichen Salzen von Verbindungen der Formel I versteht man sowohl deren organische als auch anorganische Salze, wie sie in Remington's Pharmaceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt; für basiche Gruppen unter anderen Salze mit Salzsäure, Schwefelsäure, Phosphorsäure, Carbonsäuren oder Sulfonsäuren, wie Essigsäure, Zitronensäure, Benzoesäure, Maleinsäure, Fumarsäure, Weinsäure, p-Toluolsulfonsäure.

Bevorzugt sind Verbindungen der Formel I, worin

a) X N, Y $CR^2$ und Z $CR^2$ bedeuten;

b) X $CR^2$, Y N und Z $CR^2$ bedeuten;

c) X $CR^2$, Y $CR^2$ und Z N bedeuten

oder

d) X, Y und Z jeweils N bedeuten.

Weiter sind Verbindungen der Formel I bevorzugt in welcher

a)

$R^1$

1. $(C_3-C_{10})$-Alkyl,
2. $(C_3-C_{10})$-Alkenyl,
3. $(C_3-C_{10})$-Alkinyl,
4. $(C_3-C_8)$-Cycloalkyl,
5. Benzyl,
6. Benzyl, das wie oben definiert substituiert ist oder
7. $-(CH_2)_m-B-(CH_3)_n-R^4$

bedeutet;

b)

$R^2$

1. Wasserstoff,
2. Halogen,
3. Nitro,
4. $C_v F_{2v+1}$
5. $SF_5$
6. Pentafluorphenyl
7. Cyano
8. $(C_1-C_4)$-Alkoxy, Benzyloxy
9. Phenyl,
10. Phenyl-$(C_1-C_3)$-alkyl,
11. $(C_1-C_{10})$-Alkyl,
12. $(C_3-C_{10})$-Alkenyl,
13. Phenyl-$(C_2-C_6)$-alkenyl,
14. 1-Imidazolyl-$(CH_2)_m$-

15. 1,2,3-Triazolyl-$(CH_2)_o$-
16. Tetrazolyl-$(CH_2)_m$-
17. $-(CH_2)_{o-1}-CHR^7-OR^5$
18. $-(CH_2)_o-O-COR^3$,
19. $-COR^8$,
20. $-(CH_2)_o-CO-R^8$,
21. $-S(O)_rR^6$,
22. $-CH=CH-(CH_2)_m-CHR^3-OR^6$,
23. $-CH_2=CH-(CH_2)_m-CO-R^8$,
24. $-(CH_2)_o-NH-CO-OR^9$,
25. $-(CH_2)_o-NH-SO_2-R^9$,
26. $-(CH_2)_nF$,
27. $-(CH_2)_o-SO_3R^9$,
28. $-(CH_2)_n-SO_2-NH-CO-NR^6R^9$ oder
29. einem wie unter b) 9., 10., 11., 12. oder 15. definierten Rest, der wie oben unter c) 46., 47. oder 48. jeweils wie für einen solchen Rest beschrieben definiert substituiert ist,

bedeutet;

c)
$R^8$ Wasserstoff ($C_1$-$C_5$)-Alkyl, $OR^5$, $NR^{11}R^{12}$ oder Morpholino bedeutet;

d)
T

1. eine Einfachbindung,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$,
12. -CH=CH-, oder
13.

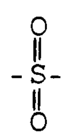

bedeutet und die übrigen Reste und Variablen wie oben definiert sind.

Besonders bevorzugt sind Verbindungen der Formel I in welcher

a)
$R^1$ ($C_3$-$C_7$)-Alkyl, ($C_3$-$C_7$)-Alkenyl oder ($C_3$-$C_7$)-Alkinyl bedeutet;

b)
$R^2$

1. Wasserstoff,
2. Chlor,
3. Brom,
4. $C_vF_{2v+1}$ mit v = 1,2 oder 3,
5. Pentafluorphenyl,
6. ($C_1$-$C_4$)-Alkoxy, Benzyloxy,
7. $-S(O)_rR^6$,
8. $SF_5$,
9. $(CH_2)_{o-1}-CHR^7-OR^5$,
10. $(CH_2)_o-O-CO-R^3$,
11. $-COR^8$,

12. $-(CH_2)_o-CO-R^8$,
13. $-CH_2-NH-CO-R^8$,
14. $-(CH_2)_o-NH-SO_2-R^9$,
15. $-CH=CH-CHR^3-OR^6$,
16. Tetrazolyl-$(CH_2)_m$-
17. $-(CH_2)_nSO_2-NH-CO-NR^6R^9$,
18. $-(CH_2)_o-SO_3R^9$ oder
19. gegebenenfalls durch Hydroxy substituiertes $(C_1-C_6)$-Alkyl, vorzugsweise Hydroxymethyl

bedeutet;

c)
$R^3$ Wasserstoff oder $(C_1-C_4)$-Alkyl bedeutet;

d)
$R^6$ Wasserstoff, $(C_1-C_4)$-Alkyl, $(C_1-C_4)$-Alkanoyl Phenyl, das gegebenenfalls mit 1 oder 2 gleichen oder verschiedenen Resten aus der Reihe Halogen, Hydroxy, Methoxy, Nitro, Cyano, $CO_2R^3$ und Trifluormethyl, substituiert sein kann oder $(C_1-C_9)$-Heteroaryl, das auch teilweise oder vollständig hydriert sein kann, vorzugsweise 2-Pyrimidyl, bedeutet;

e)
$R^7$ Wasserstoff, $(C_1-C_4)$-Alkyl, $(C_1-C_9)$-Heteroaryl, oder $(C_6-C_{12})$-Aryl-$(C_1-C_4)$-Alkyl bedeutet;

f)
$R^8$ Wasserstoff, $(C_1-C_4)$-Alkyl, $OR^5$ oder Morpholino bedeutet;

g)
$R^9$ $CF_3$, $(C_1-C_6)$-Alkyl oder Phenyl bedeutet;

h)
A

α) einen $(C_6-C_{10})$-Arylrest oder
β) $(C_1-C_4)$-Heteroaryl, der entweder aromatisch teilhydriert oder vollständig hydriert sein kann oder
γ) den Rest eines anellierten Heterobicyclus mit 8 bis 10 Ringatomen, wovon bis zu 9 Ringatome C-Atome sind, bedeutet;

wobei
A jeweils mit bis zu 3 gleichen oder verschiedenen Resten aus der Reihe Halogen, Nitro, Cyano, Hydroxy, $-NR^6R^7$, Phenyl, $-S(O)_rR^6$ und $-CO_2R^3$ substituiert sein kann;

I)
T eine Einfachbindung, -O-, -CO-, -NHCO-, $-OCH_2-$ oder

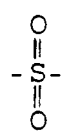

bedeutet

und die übrigen Reste und Variablen wie oben definiert sind.

Ganz besonders bevorzugt sind Verbindungen der Formel I, in welcher

a)
$R^1$ $(C_3-C_5)$-Alkyl,

b)
$R^2$ Wasserstoff, Chlor, Methoxy, $-S(O)_rR^6$ oder Benzyloxy,

c)
$R^3$ Wasserstoff oder $(C_1-C_4)$-Alkyl bedeuten,

d)
$R^4$ und $R^5$ gleich oder verschieden sind und Wasserstoff oder $(C_1-C_4)$-Alkyl bedeuten,

e)
$R^6$ Wasserstoff, $(C_1-C_4)$-Alkyl, Phenyl oder 4-Tolyl

f)
$R^{14}$ -COOH, $-PO_3H_2$, $-SO_3H$ oder 5-Tetrazolyl

g)
A Phenyl oder den Rest eines anellierten Heterocyclus mit 8 bis 10 Ringatomen, wovon bis zu 9 Ringatome C-Atome sind, bedeutet, wobei
A jedoch mit bis zu 2 gleichen oder verschiedenen Resten aus der Reihe Halogen, Nitro, Cyano, Phenyl, $-S(O)_rR^6$ und $-CO_2R^3$ substituiert sein kann,
h)
B O, NH oder S bedeutet,
i)
L $-CH_2-$ bedeutet,
j)
r 0, 1 oder 2 ist,
k)
q 0 ist,
l)
T für eine Einfachbindung steht,
m)
m 0, 1, 2 oder 3 und
n)
n 1, 2 oder 3
bedeutet und X, Y, Z wie oben definiert sind.

Die Erfindung betrifft auch ein Verfahren zur Herstellung von Verbindungen der Formel I, das dadurch gekennzeichnet ist, daß man Verbindungen der Formel II

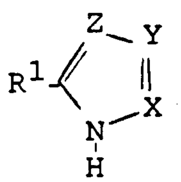

(II)

worin $R^1$, X, Y und Z wie oben definiert sind, alkyliert mit Verbindungen der Formel III

U-L-A-T-E (III)

worin L, A, T, E wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

Geeignete Fluchtgruppen U sind vorzugsweise eine nucleofuge Gruppe (vgl. Angew. Chem. 72 [1960] 71) wie Halogen, p-Toluolsulfonat, Mesylat oder Triflat.

Verfahren zur Herstellung der Vorstufen der Formel II sind u.a. bekannt aus US-Patent 4 355 044, EP-A-324 377 und EP-A-323 841.

Weitere Verfahren sind beschrieben in G. L'abbe, Chem. Rev. 69, 345 (1969); T. Srodsky in "The Chemistry of the Azido Group", Wiley, New York, 1971, S. 331; H. Wamhoff in "Comprehensive Heterocyclic Chemistry, S. Katritzky Ed., Pergamon Press, New York (1984).

Zur Alkylierung der Azole der Formel II sind z.B. entsprechnede Benzylhalogenide, -tosylate, -mesylate oder Triflate oder ensprechende Alkylhalogenide, -tosylate, mesylate oder-triflate geeignet.

Die Synthese von Derivaten wie Benzofurane, Benzthiophene und Indole mit benzylischer $-CH_3$-Gruppe wurde unter anderem vom R.P. Dickson et al. in J. Med. Chem. 29, 1637 (1986), und ibid. 29, 1643 (1986) beschrieben. Die Darstellung von Benzimidazolen, Benzothiazolen, Benzodiazinen, Benzopyronen, Benzothiazolonen, Benzotriazinen, Benzoxazinen, Benzoxazolen ist in der oben zitierten Ausgabe "Comprehensive Heterocyclic Chemistry", S. Katritzky Ed. Pergamon Press, New York (1984) skizziert.

Die Alkylierung erfolgt nach prinzipiell bekannten Verfahren in analoger Weise.

Das Azol-Derivat der Formel II wird z.B. in Gegenwart einer Base metalliert. Bevorzugte Basen sind Metallhydride der Formel MH wie Z.B. Lithium-, Natrium- oder Kaliumhydrid in beispielsweise DMF oder DMSO als Lösungsmittel oder Metallalkoxide der Formel MOR, wobei R für Methyl, Ethyl, t-Butyl steht und die Reaktion in dem entsprechenden Alkohol, DMF oder DMSO durchgeführt wird. Die so gebildeten Salze der Azole werden in einem aprotischen Lösungsmittel wie DMF oder DMSO gelöst und mit einer geeigneten Menge Alkylierungsreagenz versetzt.

Eine alternative Möglichkeit zur Deprotonierung der Azol-Derivate stellt z.B. die Umsetzung mit Kaliumcarbonat in DMF oder DMSO dar.

Die Umsetzungen werden bei Temperaturen unterhalb Raumtemperatur bis zum Siedepunkt des Reaktionsgemisches, vorzugsweise zwischen +20 °C und dem Siedepunkt des Reaktionsgemisches während etwa 1 bis 10 Stunden durchgeführt.

Die Synthese von Fragmenten der Formel II

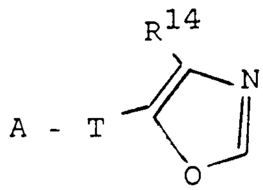

(II)

erfolgt über die Addition metallierter Isonitrile (Angew. Chem. 86, 878 (1974), 89, 351 (1977) an Säurechloride der Formel III in inerten Lösungsmitteln bei -78 °C bis zum Siedepunkt des Lösungsmittels, bevorzugt in Diethylether oder THF bei -78 °C bis 0 °C.

A - T - CO-Cl (III)

Die Synthese von Fragmenten der Formeln IV und V

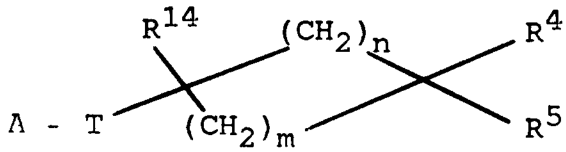

(IV)

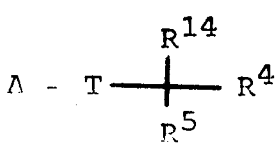

(V)

erfolgt über prinzipiell bekannte Alkylierung von Arylessigsäurederivaten oder Arylacetonitrilen in analoger Weise entweder in Ethern wie Diethylether, THF oder Dimethoxyethan mit LDA bei - 78 °C oder mit KOtBu bei Raumtemperatur in t-Butanol.

Die erfindungsgemäßen Verbindungen der Formel I haben antagnoistische Wirkung auf Angiotensin-II-Rezeptoren und können deshalb zur Behandlung der Angiotensin-II-abhängige Hypertension verwendet werden. Anwendungsmöglichkeiten bestehen weiterhin bei Herzinsuffizienz, Kardioprotektion, Myocardinfarkt, Herz-Hypertrophie, Arteriosklerosis, Nephropahtie, Nierenversagen sowie vasculären Erkrankungen des Gehirn wie transitorischen ischämischen Attacken und Gehirnschlag.

Renin ist ein proteolytisches Enzym aus der Klasse der Aspartyl-proteasen, welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderen Stimulation des des Phosphoinositol-Umsatzes ($Ca^{2+}$-Freisetzung, Aktivierung der Proteinkinase C) und Facilitation von cAMP abhängigen Hormon-Rezeptoren.

Die Affinität der Verbindungen der Formel I zum Angiotensin-II-Rezeptor kann durch Messung der $^{125}$J-Angiotensin-II- oder $^{3}$H-Angiotensin-II-Verdrängung von Rezeptoren an Zona glomerulosa Membranen von Rindernebennieren bestimmt werden. Dazu werden die präparierten Membranen in Puffer bei pH 7.4

suspendiert. Um die Degradierung des Radioliganden während der Inkubierung zu verhindern, wird Aprotinin, ein Peptidase Inhibitor, zugesetzt. Zusätzlich werden ca. 14000 cpm eines Tracers mit spezifischer Aktivität von 74 TBq/mmol (käuflich bei Amersham Buchler) und eine Menge Rezeptorprotein, die 50 % des Tracers bindet, verwendet. Die Reaktion wird durch Zugabe von 50 µl Membransuspension zu einer Mischung von 100 µl Puffer + Aprotinin; 50 µl Puffer mit oder ohne Angiotensin-II oder Rezeptorantagonist und 50 µl Tracer gestartet. Nach einer Inkubationszeit von 60 Minuten bei 25 °C werden gebundener und freier Radioligand durch einen Filtrationsassay mit Whatmann® GFIC Filtern auf einem Skatron®-Zellsammler getrennt.

Unspezifische Bindungen werden durch Behandlung der Filter mit 0,3 % Polyethylenimino pH = 10 verhindert (Sigma, Nr. 3143). Durch Messung der Radioaktivität in einem Gamma-Szintillationszähler wird die Stärke der Verdrängung des Radioliganden vom Rezeptor bestimmt. Die $IC_{50}$-Werte, welche die Konzentration des Inhibitors bedeuten, um 50 % des Liganden zu verdrängen, werden nach Chem. et. al. J. Theor. Biol. 59, 253 (1970) bestimmt. Sie liegen für die Verbindungen der Formel (I) im Bereich von $10^{-4}$-$10^{-9}$ M.

Zur Bestimmung der antagonistischen Wirkung der Verbindungen der Formel (I) kann ihr Effekt auf den Angiotensin-II-induzierten Blutdruckanstieg an narkotisierten Sprague-Dawley Ratten gemessen werden. Als Narkotikum dient Na-Thiobarbital (Trapanal®, Byk Gulden) in der Dosierung 100 mg/kg i.p. Die i.v.-Applikation erfolgt in die Vena Jugularis. Der Blutdruck wird in der A. carotis gemessen. Zunächst werden die Tiere mit Pentoliniumtartrat (10 mg/kg i.m.) vorbehandelt, so daß ein niedrigerer Blutdrucklevel erreicht wird (Ganglienblockade). ANG II (Hypertensin®, CIBA) wird im Volumen 0,1 ml/100 g in 10-minütigen Intervallen i.v. appliziert. Die Dosis beträgt 0,5 µg/kg. Die Verbindungen der Formel (I) werden in Aqua. dest. gelöst und in den Dosierungen 0,1; 1; 10 und 100 mg/kg intravenös oder intraduodenal appliziert.

Die Verbindungen der Formel (I) sind im Bereich von 0,1-100 mg/kg wirksam.

Die Erfindung bezieht sich ebenso auf pharmazeutische Zusammensetzungen bestehend aus einer Verbindung der Formel I und anderen Wirkstoffen, wie z.B. Diuretika oder nichtsteroidalen antiinflammatorischen Wirkstoffen. Die Verbindungen der Formel I können auch als Diagnostika für das Renin-Angiotensin-System verwendet werden.

Pharmazeutische Präparate enthalten eine wirksame Menge des Wirkstoffs der Formel I und eventuell andere Wirkstoffe zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht; wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Apllikation werden die aktiven Verbindungen oder deren physiologisch verträgliche Salze, gewünschtenfalls mit den dafür üblichen Substanzen wie Lösungsvermittlern, Emulgatoren oder weiterem Hilfsstoffen in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

Liste der Abkürzungen:

| | |
|---|---|
| DMF | N,H-Dimethylformamid |
| NBS | N-Bromsuccinimid |
| AIBN | $\alpha$,$\alpha$-Azobis-isobutyronitril |
| EI | electron impact |
| DCI | Desorption-Chemical Ionisation |
| RT | Raumtemperatur |
| EE | Ethylacetat (EtOAc) |
| DIP | Diisopropylether |
| THF | Tetrahydrofuran |
| Schmp. | Schmelzpunkt |

| | |
|---|---|
| MTB | Methyl-t-butylether |
| $Sdp_{xx}$ | Siedepunkt bei xx Torr |
| $Et_3N$ | Triethylamin |
| MS | Massenspektrum |
| FAB | Fast atom bombardment |
| LDA | Lithiumdiisopropylamid |

Die folgenden Beispiele veranschaulichen die Erfindung, ohne daß diese darauf beschränkt wäre.

Beispiel 1

1-[4-(1-Carboxy-1-cyclopentyl)-benzyl]-2-n-butyl-4-chlor-5-formyl-imidazol

a) 1-(p-Tolyl)-cyclopentancarbonsäure-ethylester

10 g 1-(p-Tolyl)-cyclopentancarbonsäure werden in 200 ml Ethanol suspendiert, langsam mit 4,3 ml $SOCl_2$ versetzt und 24 h am Rückfluß gekocht. Überschüssiges $SOCl_2$ wird im Vak. entfernt, mit 100 ml EE aufgenommen, 2 x mit 50 ml ges. wäßr. $Na_2CO_3$-Lösung gewaschen und über $Na_2SO_4$ getrocknet. Dann wird das Solvens i. Vak. entfernt und man erhält 11,3 g der Titelverbindung als hellbraune Kristalle Schmp.:47 ° C.

b) 1-(4-Brommethyl)-phenyl-cyclopentancarbonsäure-ethylester

2,5 g 1-(p-Tolyl)-cyclopentancarbonsäure-ethylester, 1,94 g NBS und 200 mg Benzoylperoxid werden in 50 ml Chlorbenzol 3 h lang am Rückfuß gekocht. Dann wird das Solvens i.Vak. entfernt, in 300 ml EE aufgenommen, 1 x mit 5 %iger wäßr. $Na_2SO_3$-Lösung und 2 x mit ges. wäßr. $NaHCO_3$-Lösung gewaschen und über $Na_2SO_4$ getrocknet. Schließlich wird das Solvens i. Vak. entfernt und man erhält 3,2 g der Titelverbindung als Öl.

$R_f$ ($SiO_2$; EE/Heptan 1:4) = 0,50 MS (DCI) : 311 (M + 1)

c) 1-[4-(1-Ethoxycarbonyl-1-cyclopentyl)-benzyl]-2-n-butyl-4-chlor-5-formyl-imidazol

342 mg 1-(4-Brommethyl)-phenyl-cyclopentancarbonsäure-ethylester, 187 mg 2-n-Butyl-4-chlor-5-formyl-imidazol und 138 mg $K_2CO_3$ werden in 10 ml DMF 22 h bei RT gerührt. Man verdünnt mit 100 ml EE, wäscht je 1 x mit 50 ml $H_2O$ und 5 %iger wäßr. NaCl-Lösung, trocknet über $Na_2SO_4$ und entfernt das Solvens i. Vak.. Chromatographie an Kieselgel mit DIP liefert 240 mg der Titelverbindung als farbloses Öl.

$R_f$ ($SiO_2$; DIP/MTB 1:1) = 0,68 MS (DCI) : 417 (M + 1)

d) 1-[4-(1-Carboxy-1-cyclopentyl)-benzyl]-2-n-butyl-4-chlor-5-formyl-imidazol

115 mg 1-[4-(1-Ethoxycarbonyl-1-cyclopentyl)-benzyl]-2-n-butyl-4-chlor-5-formyl-imidazol und 415 µl 1 n NaOH werden in 8 ml EtOH 29 h am Rückfluß erhitzt. Das Ethanol wird i.Vak. entfernt, mit 30 ml $H_2O$ verdünnt und mit 10 ml DIP gewaschen. Anschließend wird auf pH = 1-2 gestellt und 2 x mit je 50 ml EE extrahiert. Man trocknet über $Na_2SO_4$ und entfernt das Solvens i. Vak.. Chromatographie an Kieselgel mit MTB liefert 68 mg der Titelverbindung als hellbrauner Feststoff.

MP : 134 ° C

$R_f$ ($SiO_2$; MTB) = 0,30 MS (DCI) : 389 (M + 1)

Die Titelverbindungen der Beispiele 2 und 3 wird analog Beispiel 1 über den Methylester synthetisiert:

Beispiel 2

1-[4-(1-Carboxy-1-cyclohexyl)-benzyl]-2-n-butyl-4-chlor-5-formyl-imidazol

Beispiel 3

1-[4-(1-Carboxy-1-cyclohexyl)-benzyl]-2-n-butyl-4-methoxy-5-formyl-imidazol

670 mg 1-[4-(1-Methoxycarbonyl-1-cyclohexyl)-benzyl]-2-n-butyl-4-chlor-5-formyl-imidazol werden wie unter Beispiel 1d), beschrieben umgesetzt. Man erhält 300 mg der Titelverbindung des Beispiels 2

$R_f$ ($SiO_2$; MTB/DIP 1:1) = 0,35 MS (DCI) : 403 (M + 1)

Schmp. 147 ° C

sowie 160 mg der Titelverbindung des Beispiels 3

$R_f$ ($SiO_2$; MTB/DIP 1:1) = 0,25 MS (DCI) : 399 (M + 1)

Schmp. 167 ° C

die an Kieselgel mit MTB/DIP 1:1 chromatographisch getrennt werden.

Beispiel 4

1-(4-[1-Carboxy-1-(4,4-dimethyl)-cyclohexyl]-benzyl)-2-n-butyl-4-chlor-5-formyl-imidazol

a) 1-p-Tolyl-4,4-dimethyl-cyclohexan-carbonitril

27,6 g Kalium-t-butylat werden in 200 ml t-Butanol aufgenommen und auf Rückfluß erhitzt. Nun wird eine Lösung aus 32 g 1,5-Dibrom-3,3-dimethyl-pentan und 16,3 ml 4-Tolylacetonitril in 100 ml t-Butanol während 1 h zugetropft. 4 h wird unter Rückfluß erhitzt, das Solvens i. Vak. entfernt und der Rückstand in 200 ml ges. wäßr. $NaHCO_3$, 200 ml MTB aufgenommen. 2 x wird mit je 200 ml MTB extrahiert, über $Na_2SO_4$ getrocknet und das Solvens i. Vak. entfernt. Destillation im Feinvakuum liefert 9,7 g eines farblosen Öls.

$Sdp_{0,2}$ = 135-140 °C MS (DCI) : 228 (M + 1)

b) 1-p-Tolyl-4,4-dimethyl-cyclohexan-carbonsäure

4,0 g 1-p-Tolyl-4,4-dimethyl-cyclohexan-carbonitril und 3,0 g KOH werden in 50 ml Diethylenglycol 3 h unter Rückfluß gekocht. Man läßt abkühlen, gießt auf 100 ml 0,1 n NaOH und wäscht 2 x mit je 30 ml DIP. Dann wird mit HCl auf pH = 1-2 gestellt und 3 x mit je 100 ml Diethylether extrahiert. Über $Na_2SO_4$ wird getrocknet und das Solvens i. Vak. entfernt. Man erhält 4,0 g hellgelber Kristalle.

Schmp.: 128 °C MS (DCI) : 241 ($M^+$)

c) 1-p-Tolyl-4,4-dimethyl-cyclohexan-carbonsäure-ethylester

4,0 g 1-p-Tolyl-4,4-dimethyl-cyclohexan-carbonsäure wird in 50 ml Ethanol gelöst und mit 1,5 ml $SOCl_2$ versetzt. 3 h wird bei 50 °C, dann 3,5 h bei Rückfluß gerührt. Das Solvens wird i. Vak. entfernt, in 200 ml EE aufgenommen und 3 x mit je 50 ml ges. wäßr. $Na_2CO_3$ gewaschen. Über $Na_2SO_4$ wird getrocknet und das Solvens i. Vak. entfernt. Man erhält 5,0 g eines leicht verunreinigten, braunen Öls, das ohne Reinigung weiter eingesetzt wird.

MS (DCI) : 275 (M + 1)

d) 1-(4-Brommethyl)-phenyl-4,4-dimethyl-cyclohexan-carbonsäure-ethylester

5,0 g 1-p-Tolyl-4,4-dimethyl-cyclohexan-carbonsäure-ethylester, 2,9 g NBS und 50 mg Benzoylperoxid werden in 50 ml Chlorbenzol 1,5 h am Rückfluß gekocht. Dann wird das Solvens i. Vak. entfernt, in 200 ml EE aufgenommen, 1 x mit 100 ml ges. wäßr. $Na_2SO_3$ und 1 x mit NaCl-Lösung gewaschen. Über $Na_2SO_4$ wird getrocknet und das Solvens i. Vak. entfernt. Man erhält 5,4 g eines braunen Öls.

$R_f$ ($SiO_2$; EE/Heptan 1:8) = 0,39 MS (DCI) : 353 (M + 1)

Weitere Umsetzung erfolgt analog den Beispielen 1c und 1d zu 1-(4-[1-Carboxy-1-(4,4-dimethyl)-cyclohexyl]-benzyl)-2-n-butyl-4-chlor-5-formyl-imidazol

$R_f$ ($SiO_2$; DIP) = 0,16 MS (DCI) : 431 (M + 1)

Beispiel 5

1-(4-[1-(5-tetrazolyl)-cyclohexyl]-benzyl)-2-n-butyl-4-chlor-5-hydroxymethyl-imidazol

a) 1-(4-Tolyl)-cyclohexan-carbonitril

Die Verbindung wurde analog Beispiel 3a) synthetisiert

$Sdp_{0,3}$ = 95-100 °C MS (DCI) : 200 (M + 1)

b) 1-(4-Brommethyl-phenyl)-cyclohexan-carbonitril

Die Verbindung wurde analog Beispiel 3 d) synthetisiert.

$R_f$ ($SiO_2$; EE/Hep 1:4) = 0,46 MS (DCI) : 278 (M + 1)

c) 1-[4-(1-Cyano-1-cyclohexyl)-benzyl-2-n-butyl-4-chlor-5-formyl-imidazol

Die Verbindung wurde analog Beispiel 1c) synthetisiert.

$R_f$ ($SiO_2$; DIP) = 0,33 MS (DCI) : 384 (M + 1)

d) 1-[4-(1-Cyano-1-cyclohexyl)-benzyl]-2-n-butyl-4-chlor-5-hydroxymethyl-imidazol

1,0 g 1-[4-(1-Cyano-1-cyclohexyl)-benzyl]-2-n-butyl-4-chlor-5-formyl-imidazol und 400 mg $NaBH_4$ werden in 25 ml Ethanol gelöst und 18 h bei RT gerührt. Dann wird langsam 5 % wäßr. $NaHSO_4$-Lösung bis pH = 2 zugegeben, das Ethanol i. Vak. entfernt und 3 x mit 50 ml EE extrahiert. Über $Na_2SO_4$ wird getrocknet und das Solvens i. Vak. entfernt. Man erhält 1,0 g eines farblosen Öls.

$R_f$ ($SiO_2$; MTB) = 0,54 MS (DCI : 386 (M + 1)

e) 1-(4-[1-(2-Trimethylstannyl-5-tetrazolyl)-1-cyclohexyl]-benzyl)-2-n-butyl-4-chlor-5-hydroxymethyl-imidazol

1,1 g 1-[4-(1-cyano-1-cyclohexyl)benzyl]-2-n-butyl-4-chlor, 5-hydroxymethyl-imidazol und 1,1 g Trimethylzinnazid werden in 25 ml Toluol 48 h am Rückfluß gekocht. Das Solvens wird i. Vak. entfernt und der Rückstand ohne Reinigung weiter eingesetzt.

f) 1-(4-[1-(1-Triphenylmethyl-5-tetrazolyl)-1-cyclohexyl]-benzyl),2-n-butyl,4-chlor,5-hydroxymethyl-imidazol

Die ungereinigte Titelverbindung des Beispiels 5e) wird in 7 ml $CH_2Cl_2$ und 1,5 ml THF gelöst, mit 0,35 ml 10 n NaOH versetzt und 5 min. bei RT gerührt. Dann wird mit 900 mg Tritylchlorid versetzt und 3 Tage bei RT gerührt. Mit 100 ml EE wird verdünnt, 2 x mit je 10 ml 0,1 n NaOH und 2 x mit je 10 ml ges. wäßr. NaCl gewaschen, über $Na_2SO_4$ getrocknet und das Solvens i. Vak. entfernt. Chromatographie an Kieselgel mit MTB/DIP 1:1 liefert 400 mg der Titelverbindung als weißen Schaum.

$R_f$ ($SiO_2$; DIP/MTB 1:1) = 0,32 MS (FAB) : 677 (M + Li)

g) 1-(4-[1-(5-Tetrazolyl)-1-cyclohexyl]-benzyl)-2-n-butyl-4-chlor-5-hydroxymethyl-imidazol

400 mg 1-(4-[1-(1-Triphenylmethyl-5-tetrazolyl)-1-cyclohexyl]-benzyl)-2-n-butyl-4-chlor-5-hydroxymethyl-imidazol und 300 µl 4 n wäßr. HCl werden in 4 ml Methanol gelöst und 2 h bei RT gerührt. Dann wird mit 10 ml $H_2O$ verdünnt, das Methanol i. Vak. entfernt und mit 2 n NaOH auf pH = 13 gestellt. 3 x wird mit je 5 ml Toluol gewaschen, anschließend mit 10 % $KH_2PO_4$-Lösung auf pH = 4 gestellt und 3 x mit je 50 ml EE extrahiert. Über $Na_2SO_4$ wird getrocknet und das Solvens i. Vak. entfernt. Anschließend wird mit 20 ml EE im Ultraschallbad digeriert, abfiltriert und 2 x mit je 2 ml EE gewaschen. Der Rückstand wird im Feinvakuum getrocknet und man erhält 170 mg der Titelverbindung als farblose Kristalle.

Schmp.: 179 °C /Zers.

$R_f$ ($SiO_2$; EE/MeOH 10:1) = 0,44 MS (FAB) : 429 (M + 1)

Beispiel 6

1-[4-(4-Carboxy-5-oxazolyl)-benzyl]-2-n-butyl-4-chlor-5-formyl-imidazol

a) 4-Brommethyl-benzoesäure-methylester

50 g p-Tolylcarbonsäure-methylester, 60 g NBS und 200 mg Benzoylperoxid werden in 300 ml Chlorbenzol suspendiert und vorsichtig auf Rückfluß erwärmt. Die Reaktion setzt heftig ein. Man läßt direkt wieder abkühlen, das Chlorbenzol wird i. Vak. entfernt und der Rückstand destilliert. Man erhält 68 g der Titelverbindung als farblose Kristalle.

$Sdp_5$ = 145 °C

$R_f$ ($SiO_2$; EE/Heptan 1:4) = 0,50 MS (DCI) : 299 (M + 1)

b) 4-Brommethyl-benzoesäure

5,0 g 4-Brommethyl-benzoesäure-methylester werden in 15 ml 48 % wäßr. HBr suspendiert und 30 min. am Rückfluß gekocht. Mit $NaHCO_3$ wird auf pH = 8 gestellt und 2 x mit DIP gewaschen. Die wäßrige Phase wird dann mit $NaHSO_4$ auf pH = 2 gestellt und 3 x mit 150 ml EE extrahiert. Über $Na_2SO_4$ wird getrocknet und das Solvens i. Vak. entfernt. Man erhält 3,8 g der Titelverbindung als farblose, hygroskopische Kristalle.

Schmp.: 45 °C

$R_f$ ($SiO_2$; EE) = 0,43 MS (DCI) : 215 (M + 1)

c) 4-Brommethyl-benzoylchlorid

3,8 g 4-Brommethyl-benzoesäure wird in 10 ml $SOCl_2$ suspendiert und 1 h am Rückfluß gekocht. Überschüssiges $SOCl_2$ wird i. Vak. entfernt, der Rückstand im Feinvakuum getrocknet und roh weiter eingesetzt.

d) 5-(4-Brommethyl)-benzyl-4-methoxycarbonyl-oxazol

Das Rohprodukt aus Beispiel 5c) wird zusammen mit 5,4 ml $Et_3N$ in 50 ml wasserfreiem THF gelöst. Bei 5 °C wird 1,8 ml Isocyanessigsäure-methylester zugetropft und 3 Tage bei R.T. gerührt. Dann wird mit 300 ml EE verdünnt, 3 x mit je 100 ml 0,7 m $KH_2PO_4$, 3 x mit 100 ml ges. wäßr. $NaHCO_3$ und 1 x mit 100 ml NaCl-Lösung gewaschen, über $Na_2SO_4$ getrocknet und das Solvens i. Vak. entfernt. Chromatographie an Kieselgel mit MTB liefert 2,0 g gelber Kristalle.

Schmp.: 79 °C

$R_f$ ($SiO_2$; MTB) = 0,36

e) 1-[4-(4-Methoxycarbonyl-5-oxazolyl)-benzyl]-2-n-butyl-4-chlor-5-formyl-imidazol

296 mg 5-(4-Brommethyl)-benzyl-4-methoxycarbonyl-oxazol, 187 mg 2-n-Butyl-4-chlor-5-formyl-imidazol und 139 mg $K_2CO_3$ werden in 10 ml DMF 21 h bei RT gerührt. Dann wird mit 150 ml EE verdünnt, 2 x mit je 50 ml ges. wäßr. $NaHCO_3$-Lösung und 1 x mit 50 ml wäßr.NaCl-Lösung gewaschen, über

$Na_2SO_4$ getrocknet und das Solvens i. Vak. entfernt. Chromatographie an Kieselgel mit MTB/DIP 1:1 liefert 250 mg eines farblosen Öls.

$R_f$ ($SiO_2$; MTB/DIP 1:1) = 0,35    MS (DCI) : 402 (M + 1)

f) 1-[4-(4-Carboxy-5-oxazolyl)-benzyl]-2-n-butyl-4-chlor-5-formyl-imidazol

240 mg 1-[4-(4-Methoxycarbonyl-5-oxazolyl)-benzyl]-2-n-butyl-4-chlor-5-formyl-imidazol werden in 15 ml Methanol gelöst, mit 2,5 ml 1 n wäßr. NaOH versetzt und 20 h bei RT gerührt. Das Methanol wird i. Vak. entfernt, mit 20 ml $H_2O$ verdünnt und 2 x mit je 10 ml DIP gewaschen. Dann wird auf pH = 2 gestellt, 3 x mit je 50 ml EE extrahiert, über $Na_2SO_4$ getrocknet und das Solvens i. Vak. entfernt. Chromatographie an Kieselgel mit EE/Eisessig 10:1 liefert 90 mg eines hellgelben Schaums.

$R_f$ ($SiO_2$; EE/Eisessig 10:1) = 0,16    MS (DCI) : 388 (M + 1)

**Patentansprüche**

**1.** Verbindungen der Formel I,

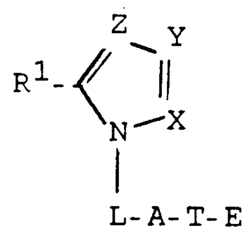

(I)

in welcher

a) X, Y und Z gleich oder verschieden sind und N oder $CR^2$ bedeuten,

b)

$R^1$

1. ($C_2$-$C_{10}$)-Alkyl,
2. ($C_3$-$C_{10}$)-Alkenyl,
3. ($C_3$-$C_{10}$)-Alkinyl,
4. $OR^3$,
5. ($C_3$-$C_8$)-Cycloalkyl,
6. ($C_4$-$C_{10}$)-Cycloalkylalkyl,
7. ($C_5$-$C_{10}$)-Cycloalkylalkenyl,
8. ($C_5$-$C_{10}$)-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, ($C_1$-$C_4$)-Alkoxy und Nitro substituiert ist,

bedeutet;

c)

$R^2$

1. Wasserstoff,
2. Halogen,
3. Nitro,
4. $C_vF_{2v+1}$,
5. $SF_5$,
6. Pentafluorphenyl,
7. Cyano,
8. ($C_1$-$C_4$)-Alkoxy, Benzyloxy,
9. Phenyl,
10. Phenyl-($C_1$-$C_3$)-alkyl,
11. ($C_1$-$C_{10}$)-Alkyl,

12. $(C_3-C_{10})$-Alkenyl,
13. Phenyl-$(C_2-C_6)$-Alkenyl,
14. 1-Imidazolyl-$(CH_2)_m$-,
15. 1,2,3-Triazolyl-$(CH_2)_n$-,
16. Tetrazolyl-$(CH_2)_m$-,
17. $-(CH_2)_{o-1}-CHR^7-OR^5$,
18. $-(CH_2)_o-O-CO-R^3$,
19. $-(CH_2)_o-S-R^6$,
20. $-S(O)_r-R^6$,
21. $-CH=CH-(CH_2)_m-CHR_3-OR^6$,
22. $-CH_2=CH-(CH_2)_m-CO-R^8$,
23. $-CO-R^8$,
24. $-CH=CH-(CH_2)_m-O-CO-R^7$,
25. $-(CH_2)_m-CH(CH_3)-CO-R^8$,
26. $-(CH_2)_o-CO-R^8$,
27.

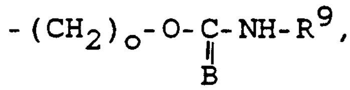

28.

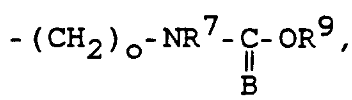

29. $-(CH_2)_o-NR^7-CO-NHR^9$,
30. $-(CH_2)_o-NR^7-SO_2R^9$,
31.

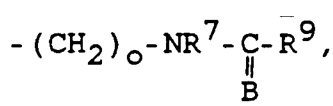

32. $-(CH_2)_nF$,
33. $-(CH_2)_n-O-NO_2$,
34. $-CH_2-N_3$,
35. $-(CH_2)_n-NO_2$,
36. $-CH=N-NR^5R^7$,
37. Phthalimido-$(CH_2)_n$-,
38.

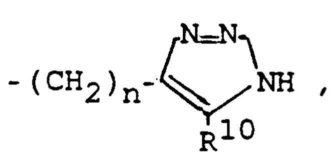

39.

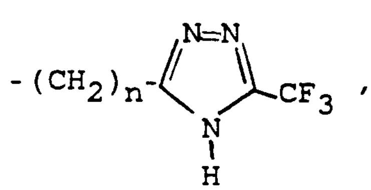

40.

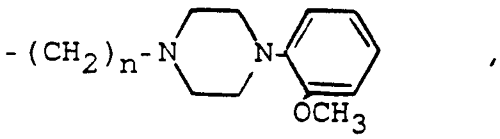

41.

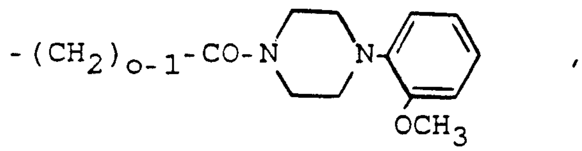

42. Phenyl-$SO_2$-NH-N = CH-,

43.

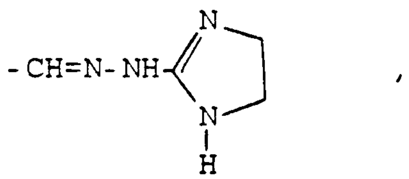

44. -$(CH_2)_n$-$SO_2$-$NR^7$-CO-$NR^6R^9$,

45. -$(CH_2)_o$-$SO_2R^9$,

46. einem wie unter c) 9. oder 10. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,

47. einem wie unter c) 11., 12. oder 20. definierten Rest, worin 1 bis alle H-Atome durch Fluor substituiert sind, oder

48. den unter c) 15. definierten Rest, der mit 1 oder 2 gleichen oder verschiedenen Resten aus der Reihe Methoxycarbonyl und ($C_1$-$C_4$)-Alkyl substituiert ist,

bedeutet;

d)

$R^3$

1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeutet;

e)

$R^4$

1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl

bedeutet;

f)

$R^5$

1. Wasserstoff,
2. ($C_1$-$C_6$)-Alkyl,

3. $(C_3-C_8)$-Cycloalkyl,
4. Phenyl oder
5. Benzyl

bedeutet;

g) $R^6$

1. Wasserstoff,
2. $(C_1-C_6)$-Alkyl,
3. $(C_3-C_8)$-Cycloalkyl,
4. $(C_6-C_{12})$-Aryl,
5. Benzyl,
6. $(C_1-C_9)$-Heteroaryl, das auch teilweise oder vollständig hydriert sein kann,
7. $(C_1-C_4)$-Alkanoyl,
8. $(C_1-C_9)$-Heteroaryl-$(C_1-C_3)$-alkyl, wobei der Heteroarylteil auch teilweise oder vollständig hydriert sein kann, oder
9. einen wie unter g) 4., 6. oder 8. definierten Rest, substituiert mit 1 oder 2 gleichen oder verschiedenen Resten aus der Reihe Halogen, Hydroxy, Methoxy, Nitro, Cyano, $CO_2R^3$, $-NR^{11}R^{12}$ und Trifluormethyl,

bedeutet;

h) $R^7$

1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl,
5. Phenyl oder
6. $(C_1-C_9)$-Heteroaryl

bedeutet;

i) $R^8$

1. Wasserstoff,
2. $(C_1-C_6)$-Alkyl,
3. $(C_3-C_8)$-Cycloalkyl,
4. Phenyl-$(CH_2)_q$-,
5. $OR^5$,
6. $NR^{11}R^{12}$ oder
7.

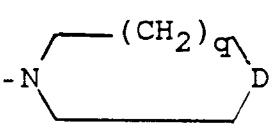

bedeutet;

j) $R^9$

1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

oder $R^6$ und $R^8$ stehen gemeinsam mit dem diese tragendem N-Atom für

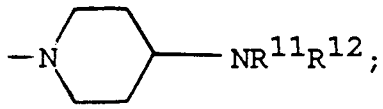

k)
$R^{10}$ Cyano, Nitro oder $CO_2R^7$ bedeutet;
l)
$R^{11}$ und $R^{12}$ gleich oder verschieden sind und
1. Wasserstoff,
2. $(C_1-C_4)$-Alkyl,
3. Phenyl,
4. Benzyl oder
5. $\alpha$-Methylbenzyl
bedeuten;
m)
D $NR^{13}$, O oder $CH_2$ bedeutet;
n)
$R^{13}$ Wasserstoff, $(C_1-C_4)$Alkyl oder Phenyl bedeutet;
o)
A
$\alpha$) einen $(C_6-C_{14})$-Arylrest oder
$\beta$) $(C_1-C_9)$-Heteroaryl, der entweder aromatisch, teilhydriert oder vollständig hydriert sein kann oder
$\gamma$) den Rest eines anellierten Heterocyclus mit 8 bis 10 Ringatomen, wovon bis zu 9 Ringatome C-Atome sind, bedeutet, wobei
A jeweils mit bis zu 3 gleichen oder verschiedenen Resten aus der Reihe
1. Halogen,
2. Oxo,
3. Nitroso,
4. Nitro,
5. Cyano,
6. Hydroxy,
7. $(C_1-C_6)$-Alkyl,
8. $(C_1-C_4)$-Alkanoyl,
9. $(C_1-C_4)$-Alkanoyloxy,
10. $CO_2R^3$,
11. Methansulfonylamino,
12. Trifluormethansulfonylamino,
13. $-CO-NH-OR^9$,
14. $-SO_2-NR^6R^7$,
15. $-CH_2-OR^7$,
16. $(C_6-C_{12})$-Aryl
17. $(C_3-C_8)$-Cycloalkyl,
18. $(C_1-C_4)$-Alkoxy,
19. $(C_1-C_9)$-Heteroaryl,
20. $CO_2R^3$,
21. $NR^6R^7$,
22. Sulfo,
23. $-SO_3R^3$,
24. $-SO_2-NR^7-CO-NR^6R^9$,
25. $-NR^7-CO-NR^6-SO_2-CH_2-R^5$,
26. $-C(CF_3)_2OH$,
27. Phosphonooxy,

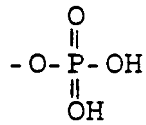 ,

28. $-PO_3H_2$,
29. $-NH-PO(OH)_2$,
30. $-S(O)_rR6$,
31. $-CO-R^8$ und
32. $-CO-NR^6R^9$

substituiert sein kann;

p)
T

1. eine Einfachbindung,
2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. $-CH=CH-$
18. $-CF=CF-$,
19. $-CH=CF-$,
20. $-CF=CH-$,
21. $-CH_2-CH_2-$,
22. $-CF_2-CF_2-$,
23. $-CH(OR^3)-$,
24. $-CH(OCOR^5)-$,
25.

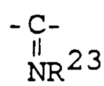 ,

26.

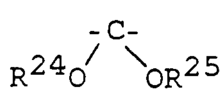

oder
27.

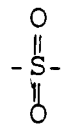

bedeutet;

q)

E einen Rest

1.

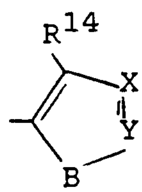

unter der Voraussetzung, daß im Falle X = Y = CH $R^{14}$ nicht COOH bedeutet,

2.

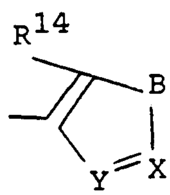

3.

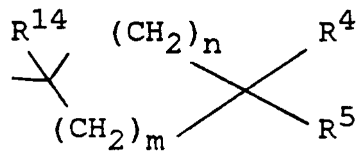

4.

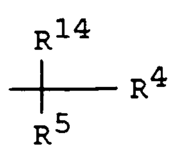

bedeutet;

r)

B O, $NR^7$ oder S bedeutet;

s)

L $(C_1\text{-}C_3)$-Alkandiyl bedeutet;

t)

$R^{14}$ $-CO_2R^3$, $-CH_2CO_2R^3$, $-PO_3H_2$, $-SO_3H$ oder Tetrazolyl bedeutet;

u)
m eine ganze Zahl von 0 bis 5 ist,
v)
n eine ganze Zahl von 1 bis 5 ist;
w)
o eine ganze Zahl von 1 bis 10 ist;
x)
r 0, 1 oder 2 ist, und
y)
v eine ganze Zahl von 1 bis 6 ist;
sowie deren physiologisch verträglichen Salze.

**2.** Verbindung der Formel I gemäß Anspruch 1, in welcher
a) X N, Y $CR^2$ und Z $CR^2$ bedeuten;
b) X $CR^2$, Y N und Z $CR^2$ bedeuten;
c) X $CR^2$, Y $CR^2$ und Z N bedeuten
oder
d) X, Y und Z jeweils N bedeuten,
sowie deren physiologisch verträglichen Salze.

**3.** Verbindung der Formel I gemäß Anspruch 1 oder 2, in welcher
a)
$R^1$
1. $(C_3-C_{10})$-Alkyl,
2. $(C_3-C_{10})$-Alkenyl,
3. $(C_3-C_{10})$-Alkinyl,
4. $(C_3-C_8)$-Cycloalkyl,
5. Benzyl,
6. Benzyl, das wie im Anspruch 1 definiert substituiert ist oder
7. $-(CH_2)_m-B-(CH_3)_n-R^4$

bedeutet;
b)
$R^2$
1. Wasserstoff,
2. Halogen,
3. Nitro,
4. $C_v F_{2v+1}$
5. $SF_5$
6. Pentafluorphenyl
7. Cyano
8. $(C_1-C_4)$-Alkoxy, Benzyloxy,
9. Phenyl,
10. Phenyl-$(C_1-C_3)$-alkyl,
11. $(C_1-C_{10})$-Alkyl,
12. $(C_3-C_{10})$-Alkenyl,
13. Phenyl-$(C_2-C_6)$-alkenyl,
14. 1-Imidazolyl-$(CH_2)_m$-
15. 1,2,3-Triazolyl-$(CH_2)_o$-
16. Tetrazolyl-$(CH_2)_m$-
17. $-(CH_2)_{o-1}-CHR^7-OR^5$
18. $-(CH_2)_o-O-COR^3$,
19. $-COR^8$,
20. $-(CH_2)_o-CO-R^8$,
21. $-S(O)_rR^6$,
22. $-CH=CH-(CH_2)_m-CHR^3-OR^6$,
23. $-CH_2=CH-(CH_2)_m-CO-R^8$,
24. $-(CH_2)_o-NH-CO-OR^9$,
25. $-(CH_2)_o-NH-SO_2-R^9$,

26. $-(CH_2)_nF$,
27. $-(CH_2)_o-SO_3R^9$,
28. $-(CH_2)_n-SO_2-NH-CO-NR^6R^9$ oder
29. einem wie unter b) 9., 10., 11., 12. oder 15. definierten Rest, der wie oben unter c) 46., 47. oder 48. jeweils wie für einen solchen Rest beschrieben definiert substituiert ist,

bedeutet;

c)
$R^8$ Wasserstoff, $(C_1-C_5)$-Alkyl, $OR^5$, $NR^{11}R^{12}$ oder Morpholino bedeutet;

d)
T

1. eine Einfachbindung,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$,
12. -CH=CH-, oder
13.

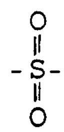

bedeutet und die übrigen Reste und Variablen wie in den vorangehenden Ansprüchen definiert sind,

sowie deren physiologisch verträglichen Salze.

**4.** Verbindung der Formel I gemäß einem der Ansprüche 1-3, in welcher

a)
$R^1$ $(C_3-C_7)$-Alkyl, $(C_3-C_7)$-Alkenyl oder $(C_3-C_7)$-Alkinyl bedeutet;

b)
$R^2$

1. Wasserstoff,
2. Chlor,
3. Brom,
4. $C_vF_{2v+1}$ mit v = 1,2 oder 3,
5. Pentafluorphenyl,
6. $(C_1-C_4)$-Alkoxy, Benzyloxy,
7. $-S(O)_rR^6$,
8. $SF_5$,
9. $(CH_2)_{o-1}-CHR^7-OR^5$,
10. $(CH_2)_o-O-CO-R^3$,
11. $-COR^8$,
12. $-(CH_2)_o-CO-R^8$,
13. $-CH_2-NH-CO-R^8$,
14. $-(CH_2)_o-NH-SO_2-R^9$,
15. $-CH=CH-CHR^3-OR^6$,
16. Tetrazolyl-$(CH_2)_m$-
17. $-(CH_2)_nSO_2-NH-CO-NR^6R^9$,
18. $-(CH_2)_o-SO_3R^9$ oder

19. gegebenenfalls durch Hydroxy substituiertes ($C_1$-$C_6$)-Alkyl, vorzugsweise Hydroxymethyl

bedeutet;

c)

$R^3$ Wasserstoff oder ($C_1$-$C_4$)-Alkyl bedeutet;

d)

$R^6$ Wasserstoff, ($C_1$-$C_4$)-Alkyl, ($C_1$-$C_4$)-Alkanoyl Phenyl, das gegebenenfalls mit 1 oder 2 gleichen oder verschiedenen Resten aus der Reihe Halogen, Hydroxy, Methoxy, Nitro, Cyano, $CO_2R^3$ und Trifluormethyl, substituiert sein kann oder ($C_1$-$C_9$)-Heteroaryl, das auch teilweise oder vollständig hydriert sein kann,

bedeutet;

e)

$R^7$ Wasserstoff, ($C_1$-$C_4$)-Alkyl, ($C_1$-$C_9$)-Heteroaryl, oder ($C_6$-$C_{12}$)-Aryl-($C_1$-$C_4$)-Alkyl bedeutet;

f)

$R^8$ Wasserstoff, ($C_1$-$C_4$)-Alkyl, $OR^5$ oder Morpholino bedeutet;

g)

$R^9$ $CF_3$, ($C_1$-$C_6$)-Alkyl oder Phenyl bedeutet;

h)

A

α) einen ($C_6$-$C_{10}$)-Arylrest oder

β) ($C_1$-$C_4$)-Heteroaryl, der entweder aromatisch teilhydriert oder vollständig hydriert sein kann oder

γ) den Rest eines anellierten Heterobicyclus mit 8 bis 10 Ringatomen, wovon bis zu 9 Ringatome C-Atome sind, bedeutet;

wobei

A jeweils mit bis zu 3 gleichen oder verschiedenen Resten aus der Reihe Halogen, Nitro, Cyano, Hydroxy, $-NR^6R^7$, Phenyl, $-S(O)_rR^6$ und $-CO_2R^3$ substituiert sein kann;

l)

T eine Einfachbindung, -O-, -CO-, -NHCO-, $-OCH_2-$ oder

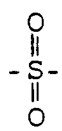

bedeutet

und die übrigen Reste und Variablen wie in dem vorangehenden Ansprüchen definiert sind, sowie deren physiologisch verträglichen Salze.

**5.** Verbindung der Formel I gemäß einem der Ansprüche 1-4, in welcher

a)

$R^1$ ($C_3$-$C_5$)-Alkyl,

b)

$R^2$ Wasserstoff, Chlor, Methoxy, Benzyloxy oder $-S(O)_rR^6$,

c)

$R^3$ Wasserstoff oder ($C_1$-$C_4$)-Alkyl bedeuten,

d)

$R^4$ und $R^5$ gleich oder verschieden sind und Wasserstoff oder ($C_1$-$C_4$)-Alkyl bedeuten,

e)

$R^6$ Wasserstoff, ($C_1$-$C_4$)-Alkyl, Phenyl oder 4-Tolyl,

f)

$R^{14}$ -COOH, $-PO_3H_2$, $-SO_3H$ oder 5-Tetrazolyl,

g)

A Phenyl oder den Rest eines anellierten Heterocyclus mit 8 bis 10 Ringatomen, wovon bis zu 9 Ringatome C-Atome sind, bedeutet, wobei

A jeweils mit bis zu 2 gleichen oder verschiedenen Resten aus der Reihe Halogen, Nitro, Cyano, Phenyl, $-S(O)_rR^6$ und $-CO_2R^3$ substituiert sein kann,

h)
B O, NH oder S bedeutet,
i)
L $-CH_2-$ bedeutet,
j)
r 0, 1 oder 2 ist,
k)
q 0 ist,
l)
T für eine Einfachbindung steht,
m)
m 0, 1, 2 oder 3 und
n)
n 1, 2 oder 3
bedeutet und X, Y, Z wie in dem vorangehenden Ansprüchen definiert sind, sowie deren physiologisch verträglichen Salze.

**6.** Verfahren zur Herstellung von Verbindungen der Formel I gemäß einem der Ansprüche 1-5, dadurch gekennzeichnet, daß man Verbindungen der Formel II

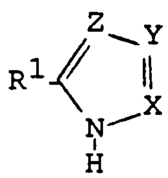

(II)

worin $R^1$, X, Y und Z wie oben definiert sind, alkyliert mit Verbindungen der Formel III

U-L-A-T-E (III)

worin L, A, T, E wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

**7.** Verbindung gemäß einem der Ansprüche 1-5 zur Anwendung als Heilmittel.

**8.** Verbindung gemäß Anspruch 7 zur Anwendung als blutdrucksenkendes Mittel.

**9.** Pharmazeutisches Mittel, enthaltend mindestens eine Verbindung gemäß einem der Ansprüche 1-5, 7 und 8.

**10.** Verfahren zur Herstellung eines Mittels gemäß Anspruch 9, dadurch gekennzeichnet, daß man eine oder mehrere Verbindungen der Formel I oder deren physiologisch verträglichen Salze zusammen mit einem physiologisch unbedenklichen Träger und gegebenenfalls weiteren Zusatz- und Hilfsstoffen in eine geeignete Darreichungsform bringt.

**Patentansprüche für folgende Vertragsstaaten : ES, GR**

**1.** Verfahren zur Herstellung einer Verbindung der Formel (I),

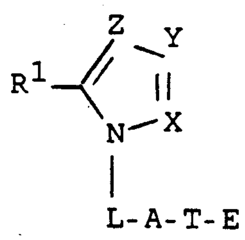

(I)

in welcher

a) X, Y und Z gleich oder verschieden sind und N oder $CR^2$ bedeuten,

b)

$R^1$

1. $(C_2\text{-}C_{10})$-Alkyl,
2. $(C_3\text{-}C_{10})$-Alkenyl,
3. $(C_3\text{-}C_{10})$-Alkinyl,
4. $OR^3$,
5. $(C_3\text{-}C_8)$-Cycloalkyl,
6. $(C_4\text{-}C_{10})$-Cycloalkylalkyl,
7. $(C_5\text{-}C_{10})$-Cycloalkylalkenyl,
8. $(C_5\text{-}C_{10})$-Cycloalkylalkinyl,
9. $-(CH_2)_m\text{-}B\text{-}(CH_2)_n\text{-}R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1\text{-}C_4)$-Alkoxy und Nitro substituiert ist,

bedeutet;

c)

$R^2$

1. Wasserstoff,
2. Halogen,
3. Nitro,
4. $C_vF_{2v+1}$,
5. $SF_5$,
6. Pentafluorphenyl,
7. Cyano,
8. $(C_1\text{-}C_4)$-Alkoxy, Benzyloxy,
9. Phenyl,
10. Phenyl-$(C_1\text{-}C_3)$-alkyl,
11. $(C_1\text{-}C_{10})$-Alkyl,
12. $(C_3\text{-}C_{10})$-Alkenyl,
13. Phenyl-$(C_2\text{-}C_6)$-Alkenyl,
14. 1-Imidazolyl-$(CH_2)_m$-,
15. 1,2,3-Triazolyl-$(CH_2)_n$-,
16. Tetrazolyl-$(CH_2)_m$-,
17. $-(CH_2)_{o\text{-}1}\text{-}CHR^7\text{-}OR^5$,
18. $-(CH_2)_o\text{-}O\text{-}CO\text{-}R^3$,
19. $-(CH_2)_o\text{-}S\text{-}R^6$,
20. $-S(O)_r\text{-}R^6$,
21. $-CH=CH\text{-}(CH_2)_m\text{-}CHR^3\text{-}OR^6$,
22. $-CH_2=CH\text{-}(CH_2)_m\text{-}CO\text{-}R^8$,
23. $-CO\text{-}R^8$,
24. $-CH=CH\text{-}(CH_2)_m\text{-}O\text{-}CO\text{-}R^7$,

25. $-(CH_2)_m-CH(CH_3)-CO-R^8$,
25. $-(CH_2)_o-CO-R^8$,
27.

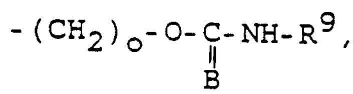

28.

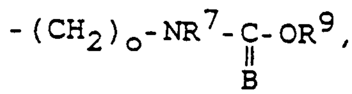

29. $-(CH_2)_o-NR^7-CO-NHR^9$
30. $-(CH_2)_o-NR^7-SO_2R^9$,
31.

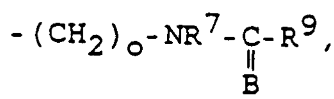

32. $-(CH_2)_nF$,
33. $-(CH_2)_n-O-NO_2$,
34. $-CH_2-N_3$,
35. $-(CH_2)_n-NO_2$,
36. $-CH=N-NR^5R^7$,
37. Phthalimido-$(CH_2)_n$-,
38.

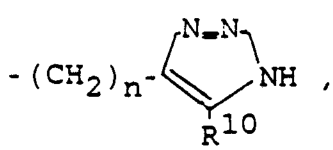

39.

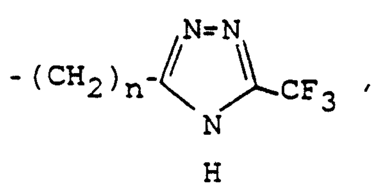

40.

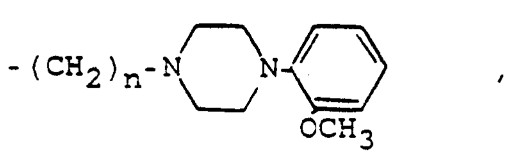

41.

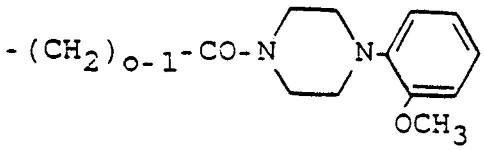

,

42. Phenyl-$SO_2$-NH-N = CH-,
43.

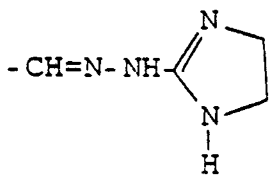

,

44. -$(CH_2)_n$-$SO_2$-$NR^7$-CO-$NR^6R^9$,
45. -$(CH_2)_o$-$SO_2R^9$,
46. einem wie unter c) 9. oder 10. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
47. einem wie unter c) 11., 12. oder 20. definierten Rest, worin 1 bis alle H-Atome durch Fluor substituiert sind, oder
48. den unter c) 15. definierten Rest, der mit 1 oder 2 gleichen oder verschiedenen Resten aus der Reihe Methoxycarbonyl und ($C_1$-$C_4$)-Alkyl substituiert ist,

bedeutet;

d)
$R^3$

1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeuten;

e)
$R^4$

1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl

bedeutet;

f)
$R^5$

1. Wasserstoff,
2. ($C_1$-$C_6$)-Alkyl,
3. ($C_3$-$C_8$)-Cycloalkyl,
4. Phenyl oder
5. Benzyl

bedeutet;

g)
$R^6$

1. Wasserstoff,
2. ($C_1$-$C_6$)-Alkyl,

3. ($C_3$-$C_8$)-Cycloalkyl,
4. ($C_6$-$C_{12}$)-Aryl,
5. Benzyl,
6. ($C_1$-$C_9$)-Heteroaryl, das auch teilweise oder vollständig hydriert sein kann,
7. ($C_1$-$C_4$)-Alkanoyl,
8. ($C_1$-$C_9$)-Heteroaryl-($C_1$-$C_3$)-alkyl, wobei der Heteroarylteil auch teilweise oder vollständig hydriert sein kann, oder
9. einen wie unter g) 4., 6. oder 8. definierten Rest, substituiert mit 1 oder 2 gleichen oder verschiedenen Resten aus der Reihe Halogen, Hydroxy, Methoxy, Nitro, Cyano, $CO_2R^3$, $-NR^{11}R^{12}$ und Trifluormethyl,

bedeutet;

h)
$R^7$
1. Wasserstoff,
2. ($C_1$-$C_6$)-Alkyl,
3. ($C_3$-$C_8$)-Cycloalkyl,
4. ($C_6$-$C_{12}$)-Aryl-($C_1$-$C_6$)-alkyl,
5. Phenyl oder
6. ($C_1$-$C_9$)-Heteroaryl

bedeutet;

i)
$R^8$
1. Wasserstoff,
2. ($C_1$-$C_6$)-Alkyl,
3. ($C_3$-$C_8$)-Cycloalkyl,
4. Phenyl-$(CH_2)_q$-,
5. $OR^5$,
6. $NR^{11}R^{12}$ oder
7.

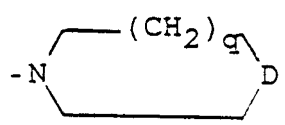

bedeutet;

j)
$R^9$
1. ($C_1$-$C_6$)-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

oder $R^6$ und $R^8$ stehen gemeinsam mit dem diese tragendem N-Atom für

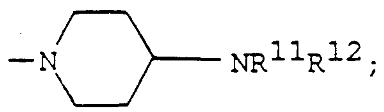

k)
$R^{10}$ Cyano, Nitro oder $CO_2R^7$ bedeutet;

l)
$R^{11}$ und $R^{12}$ gleich oder verschieden sind und
1. Wasserstoff,
2. ($C_1$-$C_4$)-Alkyl,

3. Phenyl,
4. Benzyl oder
5. $\alpha$-Methylbenzyl

bedeuten;

m)

D NR[13], O oder $CH_2$ bedeutet;

n)

$R^{13}$ Wasserstoff, $(C_1-C_4)$Alkyl oder Phenyl bedeutet;

o)

A

$\alpha$) einen $(C_6-C_{14})$-Arylrest oder

$\beta$) $(C_1-C_9)$-Heteroaryl, der entweder aromatisch, teilhydriert oder vollständig hydriert sein kann oder

$\gamma$) den Rest eines anellierten Heterocyclus mit 8 bis 10 Ringatomen, wovon bis zu 9 Ringatome C-Atome sind, bedeutet, wobei

A jeweils mit bis zu 3 gleichen oder verschiedenen Resten aus der Reihe

1. Halogen,
2. Oxo,
3. Nitroso,
4. Nitro,
5. Cyano,
6. Hydroxy,
7. $(C_1-C_6)$-Alkyl,
8. $(C_1-C_4)$-Alkanoyl,
9. $(C_1-C_4)$-Alkanoyloxy,
10. $CO_2R^3$,
11. Methansulfonylamino,
12. Trifluormethansulfonylamino,
13. $-CO-NH-OR^9$,
14. $-SO_2-NR^6R^7$,
15. $-CH_2-OR^7$,
16. $(C_6-C_{12})$-Aryl
17. $(C_3-C_8)$-Cycloalkyl,
18. $(C_1-C_4)$-Alkoxy,
19. $(C_1-C_9)$-Heteroaryl,
20. $CO_2R^3$,
21. $NR^6R^7$,
22. Sulfo,
23. $-SO_3R^3$,
24. $-SO_2-NR^7-CO-NR^6R^9$,
25. $-NR^7-CO-NR^6-SO_2-CH_2-R^5$,
26. $-C(CF_3)_2OH$,
27. Phosphonooxy,

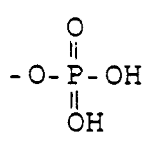 ,

28. $-PO_3H_2$,
29. $-NH-PO(OH)_2$,
30. $-S(O)_rR6$,
31. $-CO-R^8$ und
32. $-CO-NR^6R^9$

substituiert sein kann;

p)

T

1. eine Einfachbindung,
2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. -CH = CH-
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

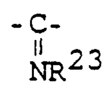 ,

26.

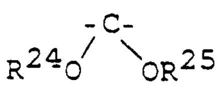

oder
27.

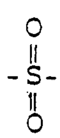

bedeutet;

q)
E einen Rest

1.

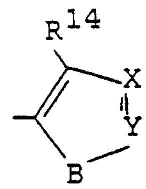

unter der Voraussetzung, daß im Falle X = Y = CH $R^{14}$ nicht COOH bedeutet,
2.

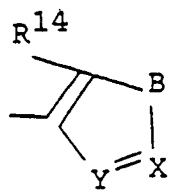

3.

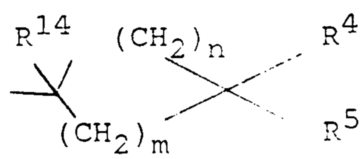

4.

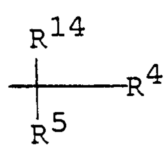

bedeutet;

r) B O, $NR^7$ oder S bedeutet;

s) L $(C_1-C_3)$-Alkandiyl bedeutet;

t) $R^{14}$ $-CO_2R^3$, $-CH_2CO_2R^3$, $-PO_3H_2$, $-SO_3H$ oder Tetrazolyl bedeutet;

u) m eine ganze Zahl von 0 bis 5 ist,

v) n eine ganze Zahl von 1 bis 5 ist;

w) o eine ganze Zahl von 1 bis 10 ist;

x) r 0, 1 oder 2 ist, und

y)

v eine ganze Zahl von 1 bis 6 ist;

sowie deren physiologisch verträglichen Salze, dadurch gekennzeichnet, daß man eine Verbindung der Formel (II)

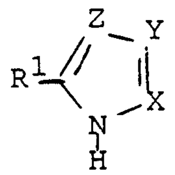

(II)

worin $R^1$, X, Y und Z wie oben definiert sind, alkyliert mit einer Verbindung der Formel (III)

U-L-A-T-E (III)

worin L, A, T, E wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

**2.** Verfahren gemäß Anspruch 1, dadurch gekennzeichnet, daß man eine Verbindung der Formel (I) herstellt, in welcher

a) X N, Y $CR^2$ und Z $CR^2$ bedeuten;

b) X $CR^2$, Y N und Z $CR^2$ bedeuten;

c) X $CR^2$, Y $CR^2$ und Z N bedeuten

oder

d) X, Y und Z jeweils N bedeuten und die übrigen Radikale wie oben definiert sind, oder

deren physiologisch verträglichen Salze.

**3.** Verfahren gemäß Anspruch 1, dadurch gekennzeichnet, daß man eine Verbindung der Formel (I) herstellt, in welcher

a)

$R^1$

1. $(C_3-C_{10})$-Alkyl,
2. $(C_3-C_{10})$-Alkenyl,
3. $(C_3-C_{10})$-Alkinyl,
4. $(C_3-C_8)$-Cycloalkyl,
5. Benzyl,
6. Benzyl, das wie im Anspruch 1 definiert substituiert ist oder
7. $-(CH_2)_m-B-(CH_3)_n-R^4$

bedeutet;

b)

$R^2$

1. Wasserstoff,
2. Halogen,
3. Nitro,
4. $C_v F_{2v+1}$
5. $SF_5$
6. Pentafluorphenyl
7. Cyano
8. $(C_1-C_4)$-Alkoxy, Benzyloxy,
9. Phenyl,
10. Phenyl-$(C_1-C_3)$-alkyl,
11. $(C_1-C_{10})$-Alkyl,
12. $(C_3-C_{10})$-Alkenyl,
13. Phenyl-$(C_2-C_6)$-alkenyl,
14. 1-Imidazolyl-$(CH_2)_m$-
15. 1,2,3-Triazolyl-$(CH_2)_o$-

16. Tetrazolyl-$(CH_2)_m$-
17. $-(CH_2)_{o-1}-CHR^7-OR^5$
18. $-(CH_2)_o-O-COR^3$,
19. $-COR^8$,
20. $-(CH_2)_o-CO-R^8$,
21. $-S(O)_rR^6$,
22. $-CH=CH-(CH_2)_m-CHR^3-OR^6$,
23. $-CH_2=CH-(CH_2)_m-CO-R^8$,
24. $-(CH_2)_o-NH-CO-OR^9$,
25. $-(CH_2)_o-NH-SO_2-R^9$,
26. $-(CH_2)_nF$,
27. $-(CH_2)_o-SO_3R^9$,
28. $-(CH_2)_n-SO_2-NH-CO-NR^6R^9$ oder
29. einem wie unter b) 9., 10., 11., 12. oder 15. definierten Rest, der wie in Anspruch 1 unter c) 46.,47. oder 48. jeweils wie für einen solchen Rest beschrieben substituiert ist,

bedeutet;

c)
$R^8$ Wasserstoff, $(C_1-C_5)$-Alkyl, $OR^5$, $NR^{11}R^{12}$ oder Morpholino bedeutet;

d)
T

1. eine Einfachbindung,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$,
12. -CH=CH-, oder
13.

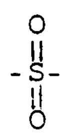

bedeutet und die übrigen Reste wie in Anspruch 1 definiert sind,

oder deren physiologisch verträglichen Salze.

**4.** Verfahren gemäß Anspruch 1, dadurch gekennzeichnet, daß man eine Verbindung der Formel (I) herstellt, in welcher

a)
$R^1$ $(C_3-C_7)$-Alkyl, $(C_3-C_7)$-Alkenyl oder $(C_3-C_7)$-Alkinyl bedeutet;

b)
$R^2$

1. Wasserstoff,
2. Chlor,
3. Brom,
4. $C_vF_{2v+1}$ mit v = 1,2 oder 3,
5. Pentafluorphenyl,
6. $(C_1-C_4)$-Alkoxy, Benzyloxy,
7. $-S(O)_rR^6$,
8. $SF_5$,
9. $(CH_2)_{o-1}-CHR^7-OR^5$,

10. $(CH_2)_o$-O-CO-$R^3$,
11. -$COR^8$,
12. -$(CH_2)_o$-CO-$R^8$,
13. -$CH_2$-NH-CO-$R^8$,
14. -$(CH_2)_o$-NH-$SO_2$-$R^9$,
15. -CH = CH-$CHR^3$-$OR^6$,
16. Tetrazolyl-$(CH_2)_m$-
17. -$(CH_2)_nSO_2$-NH-CO-$NR^6R^9$,
18. -$(CH_2)_o$-$SO_3R^9$ oder
19. gegebenenfalls durch Hydroxy substituiertes ($C_1$-$C_6$)-Alkyl, vorzugsweise Hydroxymethyl

bedeutet;

c)

$R^3$ Wasserstoff oder ($C_1$-$C_4$)-Alkyl bedeutet;

d)

$R^6$ Wasserstoff, ($C_1$-$C_4$)-Alkyl, ($C_1$-$C_4$)-Alkanoyl Phenyl, das gegebenenfalls mit 1 oder 2 gleichen oder verschiedenen Resten aus der Reihe Halogen, Hydroxy, Methoxy, Nitro, Cyano, $CO_2R^3$ und Trifluormethyl, substituiert sein kann oder ($C_1$-$C_9$)-Heteroaryl, das auch teilweise oder vollständig hydriert sein kann,
bedeutet;

e)

$R^7$ Wasserstoff, ($C_1$-$C_4$)-Alkyl, ($C_1$-$C_9$)-Heteroaryl, oder ($C_6$-$C_{12}$)-Aryl-($C_1$-$C_4$)-Alkyl bedeutet;

f)

$R^8$ Wasserstoff, ($C_1$-$C_4$)-Alkyl, $OR^5$ oder Morpholino bedeutet;

g)

$R^9$ $CF_3$, ($C_1$-$C_6$)-Alkyl oder Phenyl bedeutet;

h)

A

α) einen ($C_6$-$C_{10}$)-Arylrest oder
β) ($C_1$-$C_4$)-Heteroaryl, der entweder aromatisch teilhydriert oder vollständig hydriert sein kann oder
γ) den Rest eines anellierten Heterobicyclus mit 8 bis 10 Ringatomen, wovon bis zu 9 Ringatome C-Atome sind, bedeutet;

wobei

A jeweils mit bis zu 3 gleichen oder verschiedenen Resten aus der Reihe Halogen, Nitro, Cyano, Hydroxy, -$NR^6R^7$, Phenyl, -$S(O)_rR^6$ und -$CO_2R^3$ substituiert sein kann;

l)

T eine Einfachbindung, -O-, -CO-, -NHCO-, -$OCH_2$- oder

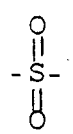

bedeutet

und die übrigen Reste wie in Anspruch 1 definiert sind, oder deren physiologisch verträglichen Salze.

**5.** Verfahren gemäß Anspruch 1, dadurch gekennzeichnet, daß man eine Verbindung der Formel (I) herstellt, in welcher

a)

$R^1$ ($C_3$-$C_5$)-Alkyl,

b)

$R^2$ Wasserstoff, Chlor, Methoxy, Benzyloxy oder -$S(O)_rR^6$,

c)

$R^3$ Wasserstoff oder ($C_1$-$C_4$)-Alkyl bedeuten,

d)

$R^4$ und $R^5$ gleich oder verschieden sind und Wasserstoff oder ($C_1$-$C_4$)-Alkyl bedeuten,

e)
$R^6$ Wasserstoff, $(C_1-C_4)$-Alkyl, Phenyl oder 4-Tolyl,
f)
$R^{14}$ -COOH, $PO_3H_2$, $-SO_3H$ oder 5-Tetrazolyl,
g)
A Phenyl oder den Rest eines anellierten Heterocyclus mit 8 bis 10 Ringatomen, wovon bis zu 9 Ringatome C-Atome sind, bedeutet, wobei
A jeweils mit bis zu 2 gleichen oder verschiedenen Resten aus der Reihe Halogen, Nitro, Cyano, Phenyl, $-S(O)_rR^6$ und $-CO_2R^3$ substituiert sein kann,
h)
B O, NH oder S bedeutet,
i)
L $-CH_2-$ bedeutet,
j)
r 0, 1 oder 2 ist,
k)
q 0 ist,
l)
T für eine Einfachbindung steht,
m)
m 0, 1, 2 oder 3 und
n)
n 1, 2 oder 3

bedeutet und X, Y, Z wie in Anspruch 1 definiert sind, oder

deren physiologisch verträglichen Salze.

**6.** Verwendung einer Verbindung hergestellt gemäß einem der Ansprüche 1-5 zur Anwendung als Heilmittel.

**7.** Verwendung einer Verbindung hergestellt gemäß einem der Ansprüche 1-5 zur Anwendung als blutdrucksenkendes Mittel.

**8.** Verfahren zur Herstellung eines pharmazeutischen Mittels, enthaltend mindestens eine Verbindung gemäß einem der Ansprüche 1-5, dadurch gekennzeichnet, daß man eine oder mehrere Verbindungen der Formel (I) oder deren physiologisch verträglichen Salze zusammen mit einem physiologisch unbedenklichen Träger und gegebenenfalls weiteren Zusatz- und Hilfsstoffen in eine geeignete Darreichungsform bringt.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 91 11 9186

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| A | EP-A-0 289 919 (BASF AG)<br>--- | | C 07 D 233/68<br>C 07 D 233/70<br>C 07 D 413/10<br>A 61 K 31/415<br>C 07 D 403/10<br>A 61 K 31/42 |
| D,A | EP-A-0 324 377 (E.I. DU PONT DE NEMOURS AND CO.)<br>----- | | |
| | | | **RECHERCHIERTE SACHGEBIETE (Int. Cl.5)** |
| | | | C 07 D<br>A 61 K |

## UNVOLLSTÄNDIGE RECHERCHE

Nach Auffassung der Recherchenahteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche: 1-10

Nicht recherchierte Patentansprüche:

Grund für die Beschränkung der Recherche:

Siehe Blatt -C-

| Recherchenort | Abschlußdatum der Recherche | Prüfer |
|---|---|---|
| DEN HAAG | 20-02-1992 | DE BUYSER I.A.F. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0409)

Grund: Die Abfassung der Ansprüche ist nicht klar und knapp zu fassen (Art. 83-84 EPA) und enthalt eine so grosse Zahl Verbindungen dass eine vollständige Recherche auf ökonomischer Gründe nicht möglich ist (Siehe Richtlinien für die Prüfung im Europaïschen Patentamt, Teil B, Kapittel III, 2. (Umfang der Recherche)). Die Recherche beschränkt sich deshalb ausschliesslich auf diejenigen Endprodukte die durch physikalische oder chemische Daten charakterisiert sind d.h. die Beispielsubstanzen.